# EUROPÄISCHE PATENTSCHRIFT

(11) **EP 2 346 474 B1**
(45) Veröffentlichungstag und Bekanntmachung des Hinweises auf die Patenterteilung: **22.05.2013**
(21) Anmeldenummer: 09752334.4
(22) Anmeldetag: 05.11.2009
(51) Int. Cl.: A61K 8/11, A61K 8/26, A61K 8/29, A61K 8/02, A61K 8/25, A61K 8/81, A61Q 5/06

(54) **PULVERFÖRMIGE STYLINGMITTEL**
POWDERY STYLING COMPOSITIONS
POUDRES DE COIFFAGE

(30) Priorität: 13.11.2008 DE 102008057261
(43) Veröffentlichungstag der Anmeldung: 27.07.2011
(73) Patentinhaber: Henkel AG & Co. KGaA, 40589 Düsseldorf (DE)
(72) Erfinder: KNAPPE, Thorsten, 22869 Schenefeld (DE); RICHTERS, Bernd, 21129 Hamburg (DE); BAYERSDÖRFER, Rolf, 22589 Hamburg (DE); KUHNERT, Oliver, 40597 Düsseldorf (DE)
(86) Internationale Anmeldenummer: PCT/EP2009/064697
(87) Internationale Veröffentlichungsnummer: WO 2010/054980

(56) Entgegenhaltungen:
- EP-A2- 0 855 177
- EP-A2- 1 457 191
- WO-A1-2006/103879
- WO-A1-2007/051511
- US-A1- 2007 055 009
- HASENZAHL S ET AL: "Fumed silica for personal care and cosmetics - versatile and effective" SOFW-JOURNAL SEIFEN, OELE, FETTE, WACHSE, VERLAG FUR CHEMISCHE INDUSTRIE, AUGSBURG, DE, 1. August 2003 (2003-08-01), Seiten 1-8, XP002289365 ISSN: 0942-7694

## Beschreibung

Die vorliegende Erfindung betrifft spezielle pulverförmige Zusammensetzungen, umfassend mindestens ein in Form von Partikeln vorliegendes Stylingpolymer sowie Kern-Hülle-Teilchen, die mindestens ein hydrophobiertes Metalloxidpulver in der Hülle und eine flüssige, wässrige Phase im Kern enthalten; die Verwendung dieser pulverförmigen Zusammensetzung zur temporären Verformung keratinischer Fasern und ein entsprechendes Verfahren.

Stylingmittel zur Verformung keratinischer Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Bekannte Formen temporärer Stylingmittel lassen sich oftmals nicht mit zufriedenstellender Genauigkeit dosieren. So lassen sich etwa Haargele, Haarcremes und Haarwachse nur noch schwierig verteilen, sobald sie einmal auf das Haar aufgebracht sind. Sobald der Kamm oder die Hände, auf die das Stylingmittel aufgebracht wurde, mit den ersten Haarpartien in Kontakt kommen, werden vergleichsweise große Mengen an Stylingmittel an das Haar abgegeben. In Haarpartien, die erst später mit dem Kamm oder den Händen erreicht werden, wird hingegen vergleichsweise wenig Stylingmittel eingearbeitet. Dies hat zur Folge, dass der Anwender entweder von Anfang an eine große Menge Stylingmittel aufbringen muss, so dass auch die zuletzt erreichten Haarpartien ausreichend Stylingmittel erhalten, oder gezwungen ist, das Stylingmittel in mehreren Schritten aufzubringen, wobei jeweils andere Haarpartien behandelt werden. Haarsprays lassen sich gleichmäßiger auf das Haar verteilen. Da der Verwender aber keine Möglichkeit hat, die Gesamtmenge an aufgebrachtem Stylingmittel visuell zu erfassen, besteht die Gefahr, dass mehr Stylingmittel auf das Haar aufgebracht wird, als eigentlich erforderlich wäre.

Pulverförmige Kosmetika sind bekannt und werden etwa im Bereich der Hautbehandlung bereits seit langem eingesetzt. Typische Beispiele sind etwa Make-up Puder oder Lidschatten. Um die pulverförmige Konsistenz zu erzielen, ist der Einsatz eines pulverförmigen Trägermaterials erforderlich. Als geeignetes Trägermaterial kann etwa ein Metalloxid, wie z.B. Siliziumdioxid verwendet werden. Besonders interessant ist hydrophobiertes Metalloxid bzw. Siliziumdioxid. Dieses kann beispielsweise ausgehend von pyrogenem Siliziumdioxid, das in verschiedenen Spezifikationen kommerziell erhältlich ist, erhalten werden. Auf der Oberfläche trägt unbehandeltes pyrogenes Siliziumdioxid Silanol- und Siloxangruppen. Dadurch hat es eine hohe Affinität zu Wasser, d.h. es ist hydrophil. Durch Umsetzung mit geeigneten organischen Siliziumverbindungen lassen sich Alkylsilylgruppen auf der Oberfläche des pyrogenen Siliziumdioxids chemisch binden. Es entstehen modifizierte Siliziumdioxidpulver, die nicht mehr von Wasser benetzt werden, d.h. die hydrophobe Eigenschaften aufweisen.

In Seifen, Öle, Fette, Wachse (SÖFW), 3 (2004), S. 4-13 wird der Einsatz von hydrophobiertem Siliziumdioxid in der Kosmetik zur Herstellung von so genanntem trockenen Wasser für die Haut beschrieben. Dabei werden die hydrophoben Eigenschaften des modifizierten Siliziumdioxids ausgenutzt, die bewirken, dass das Siliziumdioxid beim intensiven Mischen mit Wasser nicht einfach in diesem dispergiert wird. Die Wassertropfen werden vielmehr von den hydrophoben Feststoffpartikeln umhüllt und am erneuten Zusammenfließen gehindert. Auf diese Weise lassen sich pulverförmige Feststoffe mit einem Wassergehalt von bis zu über 95 % erhalten. Unter mechanischer Beanspruchung, beispielsweise beim Verreiben auf der Haut, wird das eingeschlossene Wasser wieder freigesetzt. Dieses trockene Wasser wird als Grundlage zur Herstellung von lagerstabilem, festem Wasserstoffperoxid und von verstreichbaren Zubereitungen mit sehr geringem Ölgehalt beschrieben.

Dieses Konzept liegt auch der in EP 1 235 554 B1 beschriebenen Herstellung kosmetischer oder pharmazeutischer, verflüssigbarer Pulverzusammensetzungen zu Grunde. Die Pulverzusammensetzungen umfassen hydrophob beschichtete Siliziumdioxidteilchen, in die Wasser und ein wasserlösliches Polymer eingeschlossen sind, wobei die Zusammensetzungen weniger als 1 % Öl enthalten. Durch Zugabe des wasserlöslichen Polymers soll erreicht werden, dass sich das Pulver bei der Anwendung auf der Haut angenehm und nicht kömig anfühlt, ohne dass zu diesem Zweck dem Produkt eine Ölkomponente zugegeben werden müsste. Das Polymer wird zu diesem Zweck der Wasserphase in einer Menge von 0,01 bis 5 Gew.-% zugegeben, wobei ein Gehalt an lediglich 0,1 bis 1 Gew.% bevorzugt ist. Die verflüssigbaren Pulverzusammensetzungen werden vor allem zur Herstellung dekorativer Kosmetika eingesetzt. Daneben sind auch der Einsatz in Deodorants oder Sonnenschutzmitteln, oder die Anwendung auf dem Haar als Basis von Haarbehandlungsmitteln, die Perlglanzmittel oder Pflegekomponenten enthalten, beschrieben. Eine Verwendung im Bereich der Stylingmittel ist nicht genannt.

WO 03/037287 A1 offenbart die Verwendung eines Granulats auf Basis von pyrogenem Siliziumdioxid in kosmetischen Zusammensetzungen. Die speziellen Granulate können silanisiert, d.h. hydrophobiert werden und eignen sich zur Herstellung kosmetischer Zusammensetzungen jeglicher Konsistenz, beispielsweise Flüssigkeiten, Schäume, Sprays oder Pulver. Als mögliche kosmetische Zusammensetzungen werden eine Vielzahl denkbarer Kosmetika, unter anderem HaarstylIngmittel, genannt. Dabei werden jedoch nur die üblichen Applikationsformen Lotion, Haarspray, Haarlack, Haargel und Haarwachs genannt. Ein Hinweis darauf, dass auf Basis des beschriebenen Siliziumdioxids pulverförmige Stylingmittel hergestellt werden könnten, findet sich nicht.

Die Druckschrift WO 2007/051511 A1 offenbart die Verwendung einer pulverförmigen Zusammensetzung, enthaltend 50 bis 95 Gew.-% eines wässrigen Lösungsmittels, hydrophobiertes Siliziumdioxidpulver und mindestens im wässrigen Lösemittel befindliches filmbildendes und/oder festigendes Polymer zur temporären Verformung keratinischer Fasern.

Temporare Haarumformungsmittel klassifizieren sich u.a. nach ihrem Grad der Frisurfixierung. Die pulverförmigenZusammensetzungen des Standes der Technik ermöglichen generell eine temporare Haarumformung, allerdings fehlt es Ihnen an Wirksamkeit zur Erreichung einer starken Frisurfixierung der oberen Kategorie.

Aufgabe der vorliegenden Erfindung war es daher, ein pulverformiges Haarbehandlungsmittel zur temporären Formgebung zur Verfügung zu stellen, das
- ein in der Haltbarkeit verbessertes Stylingergebnis der oberen Kategorie ergibt - insbesondere mit hervorragender Textur und Strukturgebung -
- genau und einfach dosiert werden kann,
- lagerstabil ist und
- einen minimalen Einsatz an Rohstoffen erfordert.

Überraschenderweise wurde gefunden, dass die Lehre des Standes der Technik durch das nachfolgend beschriebene Herstellverfahren für pulverförmige Zusammensetzungen verbessert wird. Die erfindungsgemäß hergestellten pulverförmigen Zusammensetzungen können sehr einfach dosiert werden. Sie lassen sich zudem sehr gleichmäßig im Haar verteilen, da die flüssige, wassrige Phase erst unter mechanischer Beanspruchung freigesetzt und eine gezielte Benetzung der Haarfasern ermöglicht wird. Das Pulver kann also zunächst vorsichtig im Haar verteilt und erst anschließend stärker mechanisch belastet werden, beispielsweise durch gezieltes Einmassieren des Pulvers in das Haar. Dadurch wird der Stylingeffekt erst direkt auf der gewünschten Haarpartie entfaltet.

Ein erster Gegenstand der vorliegenden Erfindung ist ein Verfahren zur Herstellung pulverförmiger Zusammensetzungen umfassend Kern-Hülle-Teilchen, deren Hülle Partikel mindestens eines hydrophobierten Metalloxidpulvers enthält und deren Kern eine flüssige, wässrige Phase umfasst,
worin
- eine flüssige, wässrige Phase und hydrophobiertes Metalloxidpulver unter Rühren gemischt werden und als resultierendes Gemisch eine pulverförmige Zusammensetzung umfassend Kern-Hülle-Teilchen gebildet wird, deren Hülle hydrophobiertes Metalloxidpulver enthält und deren Kern eine flüssige, wässrige Phase umfasst,
- zu der resultierenden pulverförmigen Zusammensetzung mindestens ein filmbildendes und/oder festigendes Polymer in Form eines Feststoffs zugegeben wird und unter Rühren zu einer pulverförmigen Zusammensetzung vermischt wird,
dadurch gekennzeichnet, dass das besagte filmbildende und/oder festigende Polymer ausgewählt wird aus mindestens einem Polymer der Gruppe, die gebildet wird aus Copolymeren aus Acrylsaure, (C₁ bis C₄)-Alkylacrylat, N-(C₄-Alkyl)aminoethylmethacrylat und N-(C₈-Alkyl)acrylamid.

Die Kern-Hülle-Teilchen der pulverförmigenZusammensetzung gemäß vorliegender Erfindung umfassen einen Kern, der eine wässrige, flüssige Phase umfasst. Der Kern liegt somit in flüssiger Form vor. Diesen Kern umgibt eine Hülle, die auf separierbaren einzelnen Partikeln mindestens eines hydrophobierten Metalloxidpulvers basiert.

Partikel sind im Sinne der Erfindung als Korn vorliegende Teilchen (vgl. DIN 66160: 1992-09) von Festkörpern.

Pulverförmig im Sinne der Erfindung sind Zusammensetzungen, deren Teilchen unter eigenem Gewicht frei rieselfähig sind (vgl, DIN EN ISO 6186: 1998-08).

Die verwendeten pulverförmigenZusammensetzungen enthalten hydrophobiertes Metalloxid. Die Art des hydrophobierten Metalloxids ist nicht prinzipiell beschränkt, solange sichergestellt ist, dass beim intensiven Vermischen mit der flüssigen, wässrigen Phase ein pulverförmiges Produkt entsteht. Unter hydrophobiert sind im Sinne der Erfindung solche Metalloxide zu verstehen, die zumindest an der Oberfläche der Partikel derart modifiziert wurden, dass das modifizierte Teilchen weniger von Wasser benetzt wird als das nicht modifizierte Teilchen. Insbesondere sind silanisierte, hydrophobierte Metalloxide bevorzugt. Als Reagenz zur Silanisierung des Matalloxids eignet sich erfindungsgemäß bevorzugt mindestens ein Vertreter der Gruppe, die gebildet wird, aus Silanen, Halogensilanen, Alkoxysilanen und Silazanen.

Bevorzugt geeignete hydrophobierte Metalloxide des hydrophobierten Metalloxidpulvers werden erfindungsgemäß ausgewählt aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid.

Besonders bevorzugte Aluminiumsilikate (auch Alumosilikate genannt) werden ausgewählt unter Phyllosilikaten, Tectosilikaten oder Gemischen daraus.

Bevorzugt geeignete Phyllosilikate werden ausgewählt unter Kaolinen (hier insbesondere unter Kaolinit, Dickit, Hallosit sowie Nakrit), Serpentin, Talk, Pyrophyllit, Montmorillonit, Quarz, Bentonit, Glimmer (hier insbesondere unter Illit, Muscovit, Paragonil, Phlogopil, Biotit, Lepidolith, Margarit, Smektit (hier insbesondere unter Montmorrilionit, Saponit, Nontronit, Hectorit)).

Bevorzugt geeignete Tectosilikate werden ausgewählt unter Feldspatmineralien (insbesondere Albit, Orthoklas, Anorthit, Leucit, Sodalith, Hauyn, Labradorit, Lasurit, Nosean, Nephelin), Zeolithen.

Die pulverförmigen Zusammensetzungen enthalten das hydrophobierte Metalloxidpulver vorzugsweise In einer Menge von 0,5 bis 30 Gew.-%, bezogen auf das Gewicht der gesamten pulverförmigenZusammensetzung.

Ferner hat es sich als bevorzugt erwiesen, wenn die hydrophobierten Metalloxide einen Partikeldurchmesser von weniger als 5 µm, bevorzugt weniger als 1 µm, besonders bevorzugt zwischen 20 und 100 nm, aufweisen

Besonders bevorzugt enthält die erfindungsgemäße hergestellte pulverförmige Zusammensetzung als hydrophobiertes Metalloxidpulver mindestens ein hydrophobiertes Siliziumdioxid, insbesondere mindestens ein silanisiertes, hydrophobiertes Siliziumdioxid.

Als Reagenz zur Silanisierung des Siliziumdioxids eignen sich erfindungsgemäß bevorzugt mindestens ein Vertreter der Gruppe, die gebildet wird, aus Silanen, Halogensilanen, Alkoxysilanen und Silazanen.

Bevorzugte Vertreter der Gruppe der Silane sind Hexa(C₁-C₂₀)alkyldisilane, insbesondere Hexamethyldisilan.

Wenn ein Halogensilan als Silylierungsmittel Anwendung findet, wird als bevorzugtes Halogensilan mindestens eine Verbindung aus der Gruppe ausgewählt, die gebildet wird, aus den Verbindungen

[(C₁-C₂₀)Alkyl]₂SiX_{(4,z)}

X₃Si[(CH₂)ₙ-R]

X₂[(C₁-C₂₀)Alkyl]Si(CH₂)ₙ-R

[(C₁-C₂₀)Alkyl](_{y'+1)}[R-(CH₂)ₙ]_{(2-y')}SIX

worin
X ein Chlor-, Brom- oder lodatom bedeutet,
z' eine Zahl 1, 2 oder 3 ist,
y' eine Zahl 0, 1 oder 2 ist
n eine ganze Zahl von 1 bis 20 ist und
R steht für einen Rest aus
(C₁-C₁₀)Alkyl-,Aryl-, (C₁-C₆)Perfluoroalkyl-, -NH₂, -N₃, -SCN, -CH=CH₂,
-O(O)C-C(CH₃)=CH₂, -OCH₂-CH=CH₂, 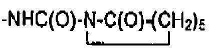 -NH-C(O)O-Me, -NH-C(O)O-Et, -NH-(CH₂)₃-Si(O(C₁-C₆)alkyl)₃.

Wenn ein Alkoxysilan als Silylierungsmittel verwendet wird, wird als bevorzugtes Alkoxysilan mindestens eine Verbindung aus der Gruppe ausgewählt, die gebildet wird, aus den Verbindungen
[(C₁-C₂₀)AlkylO]_{z}Si(C₁-C₂₀)Alkyl(_{4-z})
((C₁-C₂₀)AlkylO]_{z}Si[(CH₂)ₙ-R]_{(4-z)}
[(C₁-C₂₀)AlkylO]₂[(C₁-C₂₀)Alkyl]Si(CH₂)ₙ-R
[(C₁-C₂₀)AlkylO]₂[(C₁-C₂₀)Alkyl]₂Si(CH₂)ₙ-R
[(C₁-C₂₀)AlkylO]₂[(C₁-C₂₀)Alkyl]Si(CH₂)ₙ-R]₂
[(C₁-C₂₀Alkyl)₃SiO-C(CH₃)=N-Si(C₁-C₂₀)Alkyl₃,
worin
n eine ganze Zahl von 1 bis 20 ist und
z eine Zahl 1, 2, oder 3 bedeutet
R steht für einen Rest aus
(C₁-C₂₀)Alkyl-, Aryl-, (C₁-C₆)Perfluoroalkyl-, -NH₂, -N₃, -SCN, -CH=CH₂,
-O(O)C-C(CH₃)=CH₂, -OCH₂-CH=CH₂, 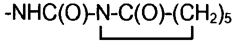 -NH-C(O)O-Me, -NH-C(O)O-Et, -NH-(CH₂)₃-Si(O(C₁-C₆)alkyl)₃.

Als bevorzugtes Silazan wird mindestens eine Verbindung aus der Klasse der Disilazane, insbesondere mindestens eine Verbindung aus Disilazanen der Formel

R'₂R"Si-NH-SiR'₂R"

ausgewählt, worin
R' eine (C₁-C₂₀)Alkylgruppe bedeutet und
R" eine (C₁-C₂₀)Alkylgruppe oder eine Vinylgruppe bedeutet. Ein besonders bevorzugtes Silazan ist Hexamethyldisilazan.

Alle oben genannten Alkylgruppen, ob (C₁-C₆)Alkyl, (C₁-C₁₀)Alkyl oder (C₁-C₂₀)-Alkyl, können sowohl zyklisch, als auch linear bzw. verzweigt sein. Beispiele für erfindungsgemäß verwendbare Alkylgruppen sind Methyl, Ethyl, n-Propyl, Isopropyl, n-Butyl, Cyclopentyl, Cyclohexyl, n-Decyl, Lauryl, Myristyl, Cetyl, Stearyl, Isostearyl und Behenyl. Ein Beispiel für eine erfindungsgemäße Arylgruppe ist die Phenylgruppe.

Beispiele für eine erfindungsgemäße (C₁-C₆)Perfluoroalkylgruppe sind Trifluormethyl, Perfluoroethyl, Perfluoropropyl und Perfluorohexyl.

Vorzugsweise werden hydrophobierte Siliziumdioxide eingesetzt, die durch Silanisierung von pyrogenem Siliziumdioxid erhalten werden.

Silanisierte, hydrophobierte Siliziumdioxide werden insbesondere bevorzugt ausgewählt aus mindestens einer Verbindung der Gruppe, die gebildet wird aus Trimethylsilylat-beschichtetem Siliziumdioxid, Dimethylsilylat-beschichtetem Siliziumdioxid, Octylsilylat-beschichtetem Siliziumdioxid.

Dabei ist es wiederum bevorzugt das hydrophobierte Metalloxidpulver aus Silica Silylate auszuwählen. Dies sind hydrophobierte Siliziumdioxide, die der INCI-Bezeichnung Silica Silylate gehorchen.

Bevorzugt sind solche hydrophobierte Siliziumdioxide, die eine spezifische Oberfläche nach BET zwischen 10 und 400 m²/g, vorzugsweise zwischen 80 bis 300 m²/g aufweisen. Dabei sind wiederum solche hydrophobierte Siliziumdioide bevorzugt geeignet, die silanisiert sind, insbesondere Silica Silylat.

Eine Vielzahl geeigneter hydrophobierter Siliziumdioxide ist kommerziell erhältlich. Beispielhaft seien Aerosil^{®} R104 V, Aerosil^{®} R106, Aerosil^{®} R202, Aerosil^{®} R805, Aerosil^{®} R812, Aerosil^{®} R812S, Aerosil^{®} R972 und Aerosil^{®} R8200, alle Degussa, sowie HDK^{®} H2000, HDK^{®} H2050 und HDK^{®} H3004, alle Wacker, genannt.

Besonders bevorzugt werden die hydrophobierten Siliziumdioxide eingesetzt, die unter den Bezeichnungen Aerosil^{®} R202, Aerosil^{®} R812S oder Aerosil^{®} R972 erhältlich sind. Ganz besonders bevorzugt kommt das Siliziumdioxid mit der INCI-Bezeichnung Sillca Silylate zum Einsatz, das von der Firma Degussa unter der Bezeichnung Aeroslil^{®} R812S vertrieben wird.

Die pulverförmigen Zusammensetzungen enthalten das hydrophobierte Siliziumoxidpulver vorzugsweise in einer Menge von 0,5 bis 30 Gew.-%. bezogen auf das Gewicht der gesamten pulverförmigenZusammensetzung.

Die optimale Menge hängt dabei vor allem von der Hydrophobizität des eingesetzten Siliziumdioxidpulvers ab. Je hydrophober das Siliziumdioxidpulver ist, desto weniger davon wird benötigt, um ein stabiles, pulverförmiges Produkt zu erhalten.

Bevorzugt enthält die erfindungsgemäße hergestellte pulverförmige Zusammensetzung als hydrophobiertes Metalloxid mindestens ein hydrophobiertes Phyllosilikat, insbesondere mindestens einen hydrophobierten Glimmer und/oder mindestens einen hydrophobierten Talk. Dabei eignet sich wiederum mindestens ein silanisiertes, hydrophobiertes Phyllosllikat bzw. mindestens ein silanisierter hydrophobierter Glimmer und/oder mindestens ein hydrophobierter Talk bevorzugt.

Als Reagenz zur Silanisierung des Phyllosilikats bzw. des Glimmers eignen sich erfindungsgemäß bevorzugt mindestens ein Vertreter der Gruppe, die gebildet wird, aus Silanen, Halogensilanen, Alkoxysilanen und Silazanen. Für die besonders bevorzugten Reaganzien wird *mutatis mutandis* auf die Ausführungen zur Silanisierung des Siliziumdioxids verwiesen (vide *supra*)*.*

Silanisierte, hydrophobierte Glimmer werden insbesondere bevorzugt ausgewählt aus mindestens einer Verbindung der Gruppe, die gebildet wird aus Trimethylsilylat-beschichtetem Glimmer, Dimethylsilylat-beschichtetem Glimmer, Octylsilylat-beschichtetem Glimmer.

Silanisierter, hydrophobierter Talk wird insbesondere bevorzugt ausgewählt aus mindestens einer Verbindung der Gruppe, die gebildet wird aus Trimethylsilylat-beschichtetem Talk, Dimethylsilylat-beschichtetem Talk, Octylsilylat-beschichtetem Talk.

Als erfindungsgemäß einsetzbares hydrophob modifiziertes Phyllosilikat sind beispielsweise mit Triethoxycaprylsilan silanisierter, hydrophobierter Glimmer mit Triethoxycaprylsilan silanisierter, hydrophobierter Talk beispielsweise von der Firma LCW zu nennen.

Die pulverförmigen Zusammensetzungen enthalten den hydrophobierten Glimme und/oder den hydrophobierten Talk vorzugsweise in einer Menge von 0,5 bis 30 Gew.%, bezogen auf das Gewicht der gesamten pulverförmigenZusammensetzung.

Die optimale Menge hängt dabei vor allem von der Hydrophobizität des eingesetzten Siliziumdioxidpulvers ab. Je hydrophober das Siliziumdioxidpulver ist, desto weniger davon wird benötigt, um ein stabiles, pulverförmiges Produkt zu erhalten.

Unter einer flüssigen, wässrigen Phase wird eine Flüssigkeit verstanden, die mindestens 40 Gew.- %, insbesondere mindestens 65 Gew.-%, Wasser bezogen auf das Gewicht der flüssigen, wässrigen Phase im Kern, enthält.

Die flüssige, wässrige Phase enthält dabei bevorzugt ein wässriges Lösungsmittel ausgewählt aus Wasser oder einem Gemisch aus Wasser und einem C₁-C₄-Alkohol, insbesondere Ethanol. Da oberflächenaktive Substanzen und Alkohole unter Umständen jedoch hydrophobiertes Siliziumdioxid benetzen und damit die hydrophoben Eigenschaften negativ beeinflussen können, kann es je nach Art des eingesetzten hydrophobierten Siliziumdioxids notwendig sein, den Gehalt an C₁-C₄-Alkohol im wassrigen Lösungsmittel unter einer kritischen Maximalmenge zu halten.

Vorzugsweise enthält die erfindungsgemäße hergestellte pulverförmige Zusammensetzung in der flüssigen, wässrigen Phase bezogen auf das Gewicht der gesamten pulverförmigen Zusammensetzung 70 Gew.-% bis 90 Gew.%, besonders bevorzugt 80 bis 90 Gew.% eines wässrigen Lösungsmittels. Vorzugsweise wird daher als wässriges Lösungsmittel Wasser oder ein Gemisch aus Wasser und maximal 60 Gew.% C₁-C₄-Alkohol, bezogen auf das Lösungsmittelgemisch, eingesetzt. Besonders bevorzugte wässrige Lösungsmittel sind Wasser oder ein Gemisch aus Wasser und maximal 30 Gew.% C₁-C₄-Alkohol, bezogen auf das Lösungsmittelgemisch. Ganz besonders bevorzugt wird als wässriges Lösungsmittel Wasser eingesetzt.

Die erfindungsgemäße hergestellten, pulverförmigenZusammensetzungen zeichnen sich dadurch aus, dass die flüssige, wässrige Phase des Kerns durch mechanische Belastung der Kern-Hülle-Teilchen, insbesondere durch Reibung und/oder Druck, aus dem Kern-Hülle-Teilchen freigesetzt wird und sich dabei aus der pulverförmigen Zusammensetzung eine Flüssigkelt bildet. Es handelt sich somit um eine pulverförmige powder-to-liquid Zusammensetzung.

Es hat sich weiterhin als vorteilhaft erwiesen, den pH-Wert der flüssigen, wässrigen Phase bevorzugt auf einen pH-Wert von kleiner oder gleich pH 7 einzustellen. Insbesondere ist es daher besonders bevorzugt, wenn die flüssige, wässrige Phase des Kerns einen pH-Wert von 2 bis 7. bevorzugt von 4 bis 6, besitzt.

Ferner sind solche pulverförmige Zusammensetzungen erfingdungsgemäß bevorzugt geeignet, deren flüssige, wässrige Phase des Kerns zusätzlich mindestens eine organische Carbonsäure mit 2 bis 7 Kohlenstoffatomen enthält. Dabei eignen sich insbesondere Zitronensäure, Weinsäure, Äpfelsäure, Benzoesäure, Salicylsäure.

Die flüssige, wässrige Phase kann zusätzlich bevorzugt solche Zusatzstoffe enthalten, die die Oberflächenspannung der wässrigen Phase nicht signifikant herabsetzen. Daher ist es bevorzugt, wenn die erfindungsgemäßen pulverförmigenZusammensetzungen in der flüssigen, wässrigen Phase nicht mehr als 0,01 Gew.-% Tenside bezogen auf das Gewicht der pulverförmigen Zusammensetzung enthalten.

Zur Verbesserung der Haarpflege können die erfindungsgemäßen hargestellten pulverförmigen Zusammensetzungen bevorzugt mindestens einen Pflegestoff enthalten, der sich bevorzugt in der flüssigen, wässrigen Phase befindet.

Die erfindungsgemäß hergestellten pulverförmigen Zusammensetzungen können als Pflegestoff mindestens einen UV-Filter. Die erfindungsgemäß geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm) oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Bevorzugt sind solche UV-Filter, deren molarer Extinktionskoeffizient am Absorptionsmaximum oberhalb von 15000, insbesondere oberhalb von 20000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.% löslich sein). Die Verwendung wasserunlöslicher UV-Filter kann daher erfindungsgemäß bevorzugt sein.

Zwei bevorzugte UV-Filter mit kationischen Gruppen sind die als Handelsprodukte erhältlichen Verbindungen Zimtsaureamidopropyl-trimethylammoniumchlarid (Incroquat^{®}UV-283) und Dodecyldimethylaminobenzamidopropyl-dimethylammoniumtosylat (Escalol^{®} HP 610).

Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt

Die UV-Filter sind in den erfindungsgemäß hergestellten Mitteln üblicherweise in Mengen 0,01-5 Gew.-%, bezogen auf die erfindungsgemäße pulverförmige Zusammensetzung, enthalten. Mengen von 0,1-2,5 Gew.-% sind bevorzugt.

Die erfindungsgemäßen Zubereitungen enthalten als zusätzlichen Pflegestoff bevorzugt mindestens ein Vitamin, ein Provitamin, eine Vitaminvorstufe sowie eines derer Derivate.

Dabei sind erfindungsgemäß solche Vitamine, Pro-Vitamine und Vitaminvorstufen bevorzugt, die üblicherweise den Gruppen A, B, C, E, F und H zugeordnet werden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber inzwischen der Trivialname Biotin durchgesetzt hat. Biotin ist ein erfindungsgemäß ganz besonders bevorzugtes Vitamin. Durch Einsatz von Biotin in den erfindungsgemäßen Zubereitungen konnte überraschender Weise die Restrukturierung der Fasern verbessert werden, eine Strukturstabilsierung erzielt werden sowie das Reizpotential der Mittel wesentlich reduziert werden. Biotin ist in den erfindungsgemäß hergestellten pulverförmigen Zusammensetzungen bevorzugt in Mengen von 0,0001 bis 2,0 Gew.- %, insbesondere in Mengen von 0,001 bis 0,2 Gew.%, jeweils bezogen auf die erfindungsgemäß hergestellte pulverförmige Zusammensetzung enthalten.

Panthenol, Pantolacton, Pyridoxin, Derivate des Pyridoxins, Nicotinsäureamid, Biotin und Mischungen davon sind erfindungsgemäß besonders bevorzugte Pflegestoffe.

Die erfindungsgemäßen pulverförmigen Zusammensetzungen können als Pflegestoff mindestens einen Pflanzenextrakt enthalten.

Die erfindungsgemäß hergestellten pulverförmigenZusammensetzungen können als Pflegestoff mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton. Erfindungsgemäß können Proteinhydrolysate sowohl pflanzfichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorlieben können. Solche Produkte werden beispielsweise unter den Warenzelchen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex), Sericin (Pentapharm) und Kerasol^{®} (Croda) vertrieben. Erfingdungsgemäß bevorzugt Ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex). Hydrosoy^{®} (Croda), hydrolupin^{®} (Croda), Hydrosesame^{®} (Croda), Hydrofritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Selbstverständlich umfasst die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten einzusetzen.

Die Proteinhydrolysate sind in den erfindungsgemäße hergestellten pulverförmigen Zusammensetzungen In Konzentrationen von 0,01 Gew. -% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die erfindungsgemäße hergestellte pulverförmige Zusammensetzung enthalten.

Im Rahmen einer neunten bevorzugten Ausführungsform enthalten die erfindungsgemäßen Zubereltungen als Pflegestoff Ectoin oder Ectoinderivate, Allantoin, Taurin und Bisabolol enthalten.

Unter dem Begriff Aminosäure werden die stereoisomeren Formen, z.B. D- und L-Formen, folgender Verbindungen verstanden: Asparagin, Arginin, Asparaginsäure, Glutamin, Glutaminsäure. β-Alanin, γ-Aminobutyrat, N,-Acetyllysin. N₅-Acetylornitin, N_{γ}-Acetyldiaminobulyrat, N_{α}-Acetyldiaminobutyrat, Histidin, Isoleucin, Leucin, Methionin, Phenylalanin, Serin, Threonin und Tyrosin.

L-Aminosäuren sind bevorzugt. Aminosäurereste leiten sich von den entsprechenden Aminosäuren ab. Die folgenden Aminosäurereste sind bevorzugt
Gly, Ala, Ser, Thr, Val, β-Ala, γ-Aminobutyrat, Asp, Glu, Asn, Aln, N_{c}-Acetyllysin, N_{δ}-Acetylornithin, N_{γ}-Acetyldiaminobutyrat, N_{α}-Acetyldiaminobutyrat.

Die Kurzschreibweise der Aminosäuren erfolgte nach der allgemein üblichen Schreibweise. Die Di- oder Tripeptidreste sind in ihrer chemische Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Die erfindungsgemäß hergestellten pulverförmigen Zusammensetzungen enthalten diese Wirkstoffe bevorzugt in Mengen von 0,001 bis 2, insbesondere von 0,01 bis 0,5 Gew.-%, jeweils bezogen auf die erfindungsgemäße pulverförmige Zusammensetzung.

Die erfindungsgemäß hergestellten pulverförmigen Zusammensetzungen können als Pflegestoff mindestens ein Mono- bzw. Oligosaccharid enthalten. Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist.

Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersaure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Weiterhin hat es sich als vorteilhaft erwiesen, wenn die erfindungsgemäßen Zubereitungen eine erhitzte, das heißt karamellisierte Zuckerart, vorzugsweise karamellisierten Rohrzucker enthalten.

Die Mono- bzw. Oligosaccharide sind in den erfindungsgemäß hergestellten pulverförmigen Zusammensetzungen bevorzugt in einer Menge von 0,1 bis 8 Gew.-%, insbesondere 1 bis 5 Gew.-%, bezogen auf die erfindungsgemäße pulverförmige Zusammensetzung, enthalten.

Alle vorgenannten bevorzugten optionalen Pflegestoffe sind bevorzugt in der flüssigen, wässrigen Phase der erfindungsgemaß hergestellten pulverförmigen Zusammensetzungen eingearbeitet.

Ein wesentliches Merkmal der erfindungsgemäß hergestellten pulverförmigen Zusammensetzungen ist die Gegenwart mindestens eines filmbildenden und/oder festigenden Polymers in Form von Partikeln. Das bedeutet, das mindestens ein filmbildendes und/oder festigendes Polymer als partikulärer Feststoff in der erfindungsgemäßen pulverförmigen Zusammensetzung vorliegt.

Da Polymere häufig multifunktional sind, können deren Funktionen nicht immer klar und eindeutig voneinander abgegrenzt werden. Insbesondere gilt dies für filmbildende und festigende Polymere. Viele Polymere, die primär als filmbildend beschrieben werden, haben auch festigende Eigenschaften und umgekehrt. Es wird an dieser Stelle daher explizit darauf verwiesen, dass im Rahmen der vorliegenden Erfindung sowohl filmbildende als auch festigende Polymere wesentlich sind. Da beide Eigenschaften nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einer Lösung einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Die filmbildenden Polymere können synthetischen oder natürlichen Ursprungs sein.

Unter filmbildenden Polymeren werden weiterhin solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nicht-ionisch, permanent kationisch oder temporär kationisch geladen sein.

Bevorzugt geeignete in Form von Partikeln vorliegende erfindungsgemäße filmbildende und/oder festigende Polymere sind bei 25°C und einem pH-Wert von pH 7 oder kleiner in entmineralisiertem Wasser unlöslich.

Im Rahmen einer bevorzugten Ausführungsform enthält die erfindungsgemäße hergestellte pulverförmige Zusammensetzung besagte filmbildende und/oder festigende Polymere in Form von Partikeln in einer Menge von 0,01 Gew.-% bis 15 Gew.-% bezogen auf das Gewicht der gesamten pulverförmigen Zusammensetzung.

Polymere dieser Art werden ausgewählt aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.

Ein Beispiel eines besonders bevorzugt im Rahmen dieser Ausführungsform verwendbaren in Form von Partikeln vorliegenden, filmbildenden und/oder festigenden Polymers ist das unter dem Handelsnamen Amphomer^{®} von der Firma National Starch erhältliche Polymer mit der INCI-Bezeichung Octylacrylamide/Acrylates/Butylaminoethylmethacrylate Copolymer.

Im Rahmen einer erfindungsgemäß bevorzugten Ausführungsform der Erfindung befindet sich das in Form von Partikeln vorliegende filmbildende und/oder festigende Polymer bevorzugt überwiegend (größer 70 %), nahezu vollständig (zu größer 99%), ganz besonders bevorzugt vollstandig, in oder auf der Hülle der Kern-Hülle-Teilchen und/oder zwischen den einzelnen Kern-Hülle-Teilchen, d.h. außerhalb des Kerns.

Im Rahmen der vorliegenden Erfindung gelten insbesondere nachfolgende Ausführungsformen IX bis XVI und XXIII bis XXX als ganz besonders bevorzugt:
IX:
   Pulverförmige Zusammensetzung umfassend Kern-Hülle-Teilchen, deren Hülle Partikel mindestens eines silanisierten, hydrophobierten Siliziumdioxidpulvers enthält und deren Kern eine flüssige,
   wässrige Phase umfasst, dadurch gekennzeichnet dass die pulverförmige Zusammensetzung mindestens ein filmbildendes und/oder festigendes Polymer in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
X:
   Pulverförmige Zusammensetzung umfassend Kern-Hülle-Teilchen, deren Hülle Partikel mindestens eines silanisierten, hydrophoblerten Siliziumdioxidpulvers enthält und deren Kern eine flüssige. wässrige Phase umfasst, dadurch gekennzeichnet, dass die pulverförmige Zusammensetzung bezogen auf das Gewicht der Zusammensetzung
      - 0,5 Gew.-% bis 30 Gew,-% silanisiertes, hydrophobiertes Siliziumdioxidpulver
         und
      - 0,01 Gew.-% bis 15 Gew.-% mindestens eines filmbildenden und/oder festigenden Polymers in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
XI:
   Pulverförmige Zusammensetzung umfassend Kern-Hülle-Teilchen, deren Hülle Partikel mindestens eines hydrophoblerten Siliziumdioxidpulvers aus Silica Silylate enthält und deren Kern eine flüssige, wässrige Phase umfasst, dadurch gekennzeichnet, dass die pulverförmige Zusammensetzung mindestens ein filmbildendes und/oder festigendes Polymer in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
XII:
   Pulverförmige Zusammensetzung umfassend Kern-Hülle-Teilchen, deren Hülle Partikel mindestens eines hydrophobierten Siliziumdioxidpulvers aus Silica Silylate enthält und deren Kern eine flüssige, wässrige Phase umfasst, dadurch gekennzeichnet, dass die pulverförmige Zusammensetzung bezogen auf das Gewicht der Zusammensetzung
      - 0,5 Gew.-% bis 30 Gew.-% Silica Silylate
         und
      - 0,01 Gew.-% bis 15 Gew.-% mindestens eines filmbildenden und/oder festigenden Polymers in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
XIII:
   Pulverförmige Zusammensetzung umfassend Kern-Hülle-Teilchen, deren Hülle Partikel mindestens eines silanisierten, hydrophobierten Siliziumdioxidpulvers enthält und deren Kern eine flüssige, wässrige Phase umfasst, dadurch gekennzeichnet, dass die pulverförmige Zusammensetzung mindestens ein filmbildendes und/oder festigendes Polymer in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid, und die flüssige, wässrige Phase einen pH-Wert von 2 bis 7, insbesondere von 4 bis 6, besitzt.
XIV:
   Pulverförmige Zusammensetzung umfassend Kern-Hülle-Teilchen, deren Hülle Partikel mindestens eines silanisierten, hydrophobierten Siliziumdioxidpulvers enthält und deren Kern eine flüssige, wässrige Phase umfasst, dadurch gekennzeichnet, dass die pulverförmige Zusammensetzung bezogen auf das Gewicht der Zusammensetzung
      - 0,5 Gew.-% bis 30 Gew.-% silanisiertes, hydrophobiertes Sitiziumdioxidpulver
         und
      - 0,01 Gew.-% bis 15 Gew.-% mindestens eines filmbildenden und/oder festigenden Polymers in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
   und die flüssige, wässrige Phase einen pH-Wert von 2 bis 7, insbesondere von 4 bis 6, besitzt.
XV:
   Pulverförmige Zusammensetzung umfassend Kern-Hülle-Teilchen, deren Hülle Partikel mindestens eines hydrophobierten Siliziumdioxidpulvers aus Silica Silylate enthält und deren Kern eine flüssige, wässrige Phase umfasst, dadurch gekennzeichnet, dass die pulverförmige Zusammensetzung mindestens ein filmbildendes und/oder festigendes Polymer in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid, und die flüssige, wässrige Phase einen pH-Wert von 2 bis 7, Insbesondere von 4 bis 6, besitzt.
XVI:
   Pulverförmige Zusammensetzung umfassend Kern-Hülle-Teilchen, deren Hülle Partikel mindestens eines hydrophobierten Siliziumdioxidpulvers aus Silica Silylate enthält und deren Kern eine flüssige, wässrige Phase umfasst, dadurch gekennzeichnet, dass die pulverförmige Zusammensetzung bezogen auf das Gewicht der Zusammensetzung
      - 0,5 Gew.-% bis 30 Gew.-% Silica Silylate
         und
      - 0,01 Gew.-% bis 15 Gew.-% mindestens eines filmbildenden und/oder festigenden Polymers in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
   und die flüssige, wässrige Phase einen pH-Wert von 2 bis 7, insbesondere von 4 bis 6, besitzt.
XXIII:
   Pulverförmige Zusammensetzung umfassend Kern-Hülle-Teilchen, deren Hülle Partikel mindestens eines hydrophobierten Talkpulvers enthält und deren Kern eine flüssige, wässrige Phase umfasst, dadurch gekennzeichnet, dass die pulverförmige Zusammensetzung mindestens ein filmbildendes und/oder festigendes Polymer in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
XXIV:
   Pulverförmige Zusammensetzung umfassend Kern-Hülle-Teilchen, deren Hülle Partikel mindestens eines hydrophoblerten Talkpulvers enthält und deren Kern eine flüssige, wässrige Phase umfasst, dadurch gekennzeichnet, dass die pulverförmige Zusammensetzung bezogen auf das Gewicht der Zusammensetzung
      - 0,5 Gew.-% bis 30 Gew.-% hydrophobiertes Talkpulver
         und
      - 0,01 Gew.-% bis 15 Gew.-% mindestens eines filmbildenden und/oder festigenden Polymers in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
XXV:
   Pulverförmige Zusammensetzung umfassend Kern-Hülle-Teilchen, deren Hülle Partikel mindestens eines hydrophobierten Glimmerpulvers enthält und deren Kern eine flüssige, wässrige Phase umfasst, dadurch gekennzeichnet, dass die pulverförmige Zusammensetzung mindestens ein filmbildendes und/oder festigendes Polymer in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
XXVI:
   Pulverförmige Zusammensetzung umfassend Kern-Hülle-Teilchen, deren Hülle Partikel mindestens eines hydrophobierten Glimmerpulvers enthält und deren Kern eine flüssige, wässrige Phase umfasst, dadurch gekennzeichnet, dass die pulverförmige Zusammensetzung bezogen auf das Gewicht der Zusammensetzung
      - 0,5 Gew.-% bis 30 Gew.-% hydrophobiertes Glimmerpulver
         und
      - 0,01 Gew.-% bis 15 Gew.-% mindestens eines filmbildenden und/oder festigenden Polymers in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
XXVII:
   Pulverförmige Zusammensetzung umfassend Kern-Hülle-Teilchen, deren Hülle Partikel mindestens eines hydrophobierten Talkpulvers enthält und deren Kern eine flüssige, wässrige Phase umfasst, dadurch gekennzeichnet, dass die pulverförmige Zusammensetzung mindestens ein filmbildendes und/oder festigendes Polymer in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid, und die flüssige, wässrige Phase einen pH-Wert von 2 bis 7, insbesondere von 4 bis 6, besitzt.
XXVIII:
   Pulverförmige Zusammensetzung umfassend Kern-Hülle-Teilchen, deren Hülle Partikel mindestens eines hydrophobierten Talkpulvers enthält und deren Kern eine flüssige, wässrige Phase umfasst, dadurch gekennzeichnet, dass die pulverförmige Zusammensetzung bezogen auf das Gewicht der Zusammensetzung
      - 0,5 Gew.-% bis 30 Gew.-% hydrophobiertes Talkpulver
         und
      - 0,01 Gew.-% bis 15 Gew.-% mindestens eines filmbildenden und/oder festigenden Polymers in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
      und die flüssige, wässrige Phase einen pH-Wert von 2 bis 7, insbesondere von 4 bis 6, besitzt.
XXIX:
   Pulverförmige Zusammensetzung umfassend Kern-Hülle-Teilchen, deren Hülle Partikel mindestens eines hydrophobierten Glimmerpulvers enthält und deren Kern eine flüssige, wässrige Phase umfasst, dadurch gekennzeichnet, dass die pulverförmige Zusammensetzung mindestens ein filmbildendes und/oder festigendes Polymer in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(Cₐ-Alkyl)acrylamid,
   und die flüssige, wässrige Phase einen pH-Wert von 2 bis 7, insbesondere von 4 bis 6, besitzt.
XXX:
   Pulverförmige Zusammensetzung umfassend Kern-Hülle-Teilchen, deren Hülle Partikel mindestens eines hydrophobierten Glimmerpulvers enthält und deren Kern eine flüssige, wässrige Phase umfasst, dadurch gekennzeichnet, dass die pulverförmige Zusammensetzung bezogen auf das Gewicht der Zusammensetzung
      - 0,5 Gew.-% bis 30 Gew.-% hydrophobiertes Glimmerpulver
         und
      - 0,01 Gew.-% bis 15 Gew.-% mindestens eines filmbildenden und/oder festigenden Polymers in Form von Partikeln enthält, welches ausgewählt wird aus der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.
      und die flüssige, wässrige Phase einen pH-Wert von 2 bis 7, insbesondere von 4 bis 6, besitzt.

Hierbei gelten wiederum *mutatis mutandis* die genannten bevorzugten Ausführungsformen der Inhaltsstoffe bzw. bevorzugten zusätzlichen Inhaltsstoffe (*vide supra*) als bevorzugt.

Die erfindungsgemäßen pulverformigen Zusammensetzungen können in nahezu beliebigen Behältnissen konfektioniert werden. Es muss lediglich sichergestellt werden, dass die mechanische Belastung des Pulvers bei der Entnahme der Zusammensetzung nicht so hoch ist, dass berelts bei der Entnahme das Pulver in flüssige Form überführt wird. Geeignet sind beispielsweise Tiegel, Flaschen oder auch Tetrapacks. wobei das Behältnis beispielsweise mit einer Schütt- und Dosiervorrichtung ausgestaltet werden kann.

Alle erfindungsgemäß hergestellten pulverförmigen Zusammensetzungen werden durch folgendes Herstellungsverfahren bereitgestellt:
Verfahren zur Herstellung einer pulverförmigen Zusammensetzung worin
   - eine flüssige, wässrige Phase und hydrophobiertes Metalloxidpulver unter Rühren gemischt werden und als resultierendes Gemisch eine pulverförmige Zusammensetzung umfassend Kern-Hülle-Teilchen gebildet wird, deren Hülle hydrophobiertes Metalloxidpulver enthält und deren Kern eine flüssige, wässrige Phase umfasst,
   - zu der resultierenden pulverförmigen Zusammensetzung mindestens ein filmbildendes und/oder festigendes Polymer in Form eines Feststoffs zugegeben wird und unter Rühren zu einer pulverförmigen Zusammensetzung vermischt wird, dadurch gekennzeichnet. dass das besagte filmbildende und/oder festigende Polymer ausgewählt wird aus mindestens einem Polymer der Gruppe, die gebildet wird aus Copolymeren aus Acrylsäure, (C₁ bis C₄-)Alkylacrylat, N-(C₄-Alkyl)aminoethylmethacrylat und N-(C₈-Alkyl)acrylamid

Zur Herstellung der bevorzugten pulverförmigen Zusammensetzungen des ersten Erfindungsgegenstandes werden ebenso bevorzugt die bevorzugten Inhaltsstoffe des ersten Erfindungsgegenstandes (*vide supra*) in dem erfindungsgemäßen Verfahren eingesetzt.

Insbesondere die Einstellung des erfindungsgemäß bevorzugten pH-Wertes der flüssigen, wässrigen Phase erfolgt, bevor diese mit dem hydrophobierten Metalloxidpulver wie oben beschrieben gemischt wird.

Gleiches gilt für die Einarbeitung weiterer Bestandteile in die flüssige, wässrige Phase. Diese Bestandtelle bzw. Inhaltsstoffe werden in die flüssige, wässrige Phase eingearbeitet, bevor diese mit dem hydrophobierten Metalloxidpulver wie oben beschrieben gemischt wird.

Ein weiterer Gegenstand der Erfindung ist die Verwendung einer erfindungsgemäß hergestellten pulverförmigen Zusammensetzung zur temporären Verformung keratinhaltiger Fasern, insbesondere menschlicher Haare.

Unter keratinhaltigen Fasern sind dabei erfindungsgemäß Pelze, Wolle. Federn und insbesondere menschliche Haare zu verstehen.

Ein weiterer Erfindungsgegenstand Ist ein Verfahren zur temporären Verformung keratinhaltiger Fasern, insbesondere menschlicher Haare, in dem
- eine erfindungsgemäß hergestellte pulverförmige Zusammensetzung gemäß erstern Erfindungsgegenstand vor, während oder nach dem Auftragen auf die keratinhaltigen Fasern einer mechanischen Belastung ausgesetzt wird, wobei sich die pulverförmige Zusammensetzung in eine Flüssigkeit umwandelt,
- die keratinhaltigen Fasern
   - vor, nach oder wahrend des Auftragens der pulverförmigen Zusammensetzung und/oder
   - vor, nach oder während der mechanischen Belastung der pulverförmigen Zusammensetzung
   in Form gebracht werden,
- die Form der keratinhaltigen Fasern durch die in besagte Flüssigkeit umgewandelte pulverförmige Zusammensetzung temporär fixiert wird.

Unter keratinhaltigen Fasern sind dabei erfindungsgemäß Pelze, Wolle, Federn und insbesondere menschliche Haare zu verstehen.

Bei der Verwendung der pulverförmigen Zusammensetzung zur temporären Verformung keratinischer Fasern wird zunächst bevorzugt die gewünschte Menge der pulverförmigen Zusammensetzung dem Behältnis entnommem Die Zusammensetzung kann dabei direkt auf die zu behandelnde keratinische Faser oder aber beispielsweise auf die Hand gegeben werden. Im ersten Fall kann das aufgebrachte Pulver direkt auf der keratinischen Faser einer mechanischen Belastung, beispielsweise mittels der Hände, ausgesetzt werden, wodurch die flüssige, wässrige Phase direkt auf der Faser freigesetzt und die Wirkung des in Form von Partikeln vorliegenden filmbildenden und/oder festigenden Polymer entfaltet werden. Wird die pulverförmige Zusammensetzung zunächst auf die Hand gegeben, so kann es zunächst vorsichtig im Haar verteilt und wiederum erst anschließend stärker mechanisch belastet werden, beispielsweise durch gezieltes Einmassieren des Pulvers in das Haar. Dadurch wird die flüssige, wässrige Phase direkt auf der Faser freigesetzt und die Wirkung des in Form von Partikeln vorliegenden filmbildenden und/oder festigenden Polymer entfaltet. So lässt sich sehr gezielt eine hervorragende Stylingwirkung erreichen. Es ist natürlich auch möglich, die pulverförmige Zusammensetzung bereits auf der Hand zu verreiben und erst das entstehende flüssige oder pastenartige Mittel auf die keratinische Faser aufzubringen. Diese Vorgehensweise ist allerdings nicht bevorzugt, da dabei auf einen wesentlichen Vorteil der pulverförmigen Konsistenz des Stylingmittels, nämlich die gute Verteilbarkeit, verzichtet wird. Die pulverförmige Zusammensetzung lässt sich natürlich auch mit einem Hilfsmittel, etwa einem Pinsel, einem Schwamm, einem Tuch, einer Bürste oder einem Kamm auftragen.

Die nachfolgenden Beispiele sollen den Gegenstand der vorliegenden Erfindung erläutern ohne ihn in irgendeiner Weise zu beschränken.

### Beispiele

Die im Folgenden angegebenen Mengenangaben verstehen sich - soweit nichts anderes vermerkt ist - in Gewichtsprozent.

### 1. Herstellung pulverförmiger Stylingmittel

Es wurden wie nachfolgend beschrieben die pulverförmigen Stylingmittel V1, V2, E1 bis E3 hergestellt, wobei diese folgende Zusammensetzung aufwiesen:

| **Rohstoffe** | **V1** | **V2** | **E1** | **E2** | **E3** |
|---|---|---|---|---|---|
| Aerosil^{®} R 812 S | 19,00 | 19,00 | 19,00 | 19,00 | 19,00 |
| Natriumbenzoat | 0,20 | 0,20 | 0,20 | 0,20 | 0,20 |
| Zitronensäure Monohydrat | 0,02 | 0,02 | 0,02 | 0,02 | 0,02 |
| Amphomer^{®} | - | - | 0,05 | 0,10 | 10,00 |
| PVP/VA W 635^{®} | - | 0,20 | - | - | - |
| Wasser, entsalzt | ad 100 | ad 100 | ad 100 | ad 100 | ad 100 |

Die Zusammensetzungen V1 und V2 sind nicht erfindungsgemäß. Die Zusammensetzungen E1 bis E3 sind erfindungsgemäß.

Wasser, Zitronensäure Monohydrat und Natriumbenzoat (sowie für A2 PVPNA 60/40 W NP) wurden in einem Gefäß gemischt. Daraus resultierte eine Flüssigkeit mit einem pH-Wert zwischen 4,5 und 5. Diese Flüssigkeit wurde mit dem hydrophobierten Siliziumdioxidpulver Aerosil^{®} R 812 S (INCI-Bezeichnung: Silica Silylate) versetzt. Nach einer Rührzeit von jeweils 30 bis 45 Sekunden hatte sich jeweils ein stabiles Pulver gebildet. Das Amphomer (sofern enthalten) wurde als Feststoff zu dem stabilen Pulver gegeben und unter rühren untergemengt. Das so erhaltene fertige Stylingpulver wurde in Polyethylenflaschen abgefüllt.

### 2. Anwendung

Im Rahmen eines Halbseitentests wurden jeweils 6 Probanden mit den oben genannten Stylingmitteln frisiert. Dabei wurde das Kopfhaar halbseitig in eine rechte und eine linke Hälfte eingeteilt. Die linke Hälfte wurde mit dem Vergleichsstylingpulver frisiert, die rechte Hälfte wurde mit einem erfindungsgemäßen Stylingpulver frisiert. Zu diesem Zweck wurde die Menge des jeweiligen Pulvers im Haar verteilt Anschließend wurde die Zusammensetzung durch massieren und kneten verflüssigt und dabei das Haar mit den Händen in die gewünschte Form gebracht.

Je Proband wurden gleiche Mengen des Vergleichspulvers und des erfindungsgemäßen Pulvers angewendet. Folgende Kombinationen wurden verglichen: V1/E1, V1/E2, V1/E3, V2/E1, V2/E2, V2/E3.

Für die erfindungsgemäßen Zusammensetzungen E1 bis E3 wurde im Vergleich zu den Zusammensetzungen V1 und V2 ein verbessertes Stylingergebnis erhalten. Das Haar besaß deutlich mehr Struktur und Textur. Trotz des ungelösten filmbildenden und/oder festigenden Polymers Amphomer waren überraschenderweise weder Pulverrückstände im behandelten Haar sichtbar, noch wurde ein übermäßiger Mattierungseffekt festgestellt.

### 3. Verzeichnis der eingesetzten Rohstoffe

Die im Rahmen der Beispiele eingesetzten Rohstoffe sind wie folgt definiert:

| | |
|---|---|
| Aerosil^{®} R 812 S | INCI-Bezeichnung: Silica Silylate (Evonik Degussa) |
| Amphomer^{®} | amphoteres Polymer mit der INCI-Bezeichnung: Octylacrylamide/Acrylates/Butyl-aminoethyl Methacrylate Copolymer, weißes Pulver (National Starch) |
| PVP/VA W 635^{®} | Vinylpyrrolidon Vinylacetat Copolymer (60:40) (ca. 48-52% Aktivsubstanz in Wasser als klare viskose Flüssigkeit, pH 5 bis 7; INCI-Bezeichnung: VP/VA Copolymer) (ISP) |

## Patentansprüche

1. Verfahren zur Herstellung einer pulverförmigen Zusammensetzung worin
- eine flüssige, wässrige Phase und hydrophobiertes Metalloxidpulver unter Rühren gemischt werden und als resultierendes Gemisch eine pulverförmige Zusammensetzung umfassend Kern-Hülle-Teilchen gebildet wird, deren Hülle hydrophobiertes Metalloxidpulver enthält und deren Kern eine flüssige, wässrige Phase umfasst,
- zu der resultierenden pulverförmigen Zusammensetzung mindestens ein filmbildendes und/oder festigendes Polymer in Form eines Feststoffs zugegeben wird und unter Rühren zu einer pulverförmigen Zusammensetzung vermischt wird,
**dadurch gekennzeichnet, dass** das besagte filmbildende und/oder festigende Polymer ausgewählt wird aus mindestens einem Polymer der Gruppe, die gebildet wird aus Copolymeren aus Acrylsäure, (C₁ bis C₄)-Alkylacrylat, N-(C₄-Alkyl)aminoethylmethacrylat und N-(C₈-Alkyl)acrylamid.

2. Verfahren nach Anspruch 1, **dadurch gekennzeichnet, dass** das hydrophobierte Metalloxid des hydrophobierten Metalloxidpulvers ausgewählt wird aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid.

3. Verfahren nach einem der Ansprüche 1 oder 2, **dadurch gekennzeichnet, dass** hydrophobiertes Metalloxidpulver in einer Menge von 0,5 Gew.-% bis 30 Gew.-% bezogen auf das Gewicht des Verfahrensendprodukts enthalten ist.

4. Verfahren nach einem der Ansprüche 1 bis 3, **dadurch gekennzeichnet, dass** das hydrophobierte Metalloxidpulver ausgewählt wird aus Silica Silylate.

5. Verfahren nach einem der Ansprüche 1 bis 4, **dadurch gekennzeichnet, dass** die flüssige, wässrige Phase des Kerns ein wässriges Lösungsmittel in einer Menge von 70 Gew.-% bis 90 Gew.-% bezogen auf das Gewicht des Verfahrensendprodukts enthält.

6. Verfahren nach einem der Ansprüche 1 bis 5, **dadurch gekennzeichnet, dass** das besagte filmbildende und/oder festigende Polymer in Form von Partikeln in einer Menge von 0,01 Gew.-% bis 15 Gew.-% bezogen auf das Gewicht der gesamten pulverförmigen Zusammensetzung enthalten ist.

7. Verfahren nach einem der Ansprüche 1 bis 6, **dadurch gekennzeichnet, dass** die flüssige, wässrige Phase des Kerns einen pH-Wert von 2 bis 7, bevorzugt von 4 bis 6, besitzt.

8. Verfahren nach einem der Ansprüche 1 bis 7, **dadurch gekennzeichnet, dass** die flüssige, wässrige Phase des Kerns zusätzlich mindestens eine organische Carbonsäure mit 2 bis 7 Kohlenstoffatomen enthält.

9. Pulverförmige Zusammensetzung, erhältlich durch ein verfahren nach einem der Ansprüche 1 bis 8.

10. Verwendung einer pulverförmigen Zusammensetzung nach Anspruch 9 zur temporären Verformung keratinhaftiger Fasern, insbesondere menschlicher Haare.

11. Verfahren zur temporären Verformung keratinhaltiger Fasern, insbesondere menschlicher Haare, in dem
- eine pulverförmige Zusammensetzung nach Anspruch 9 vor, während oder nach dem Auftragen auf die keratinhaltigen Fasern einer mechanischen Belastung ausgesetzt wird, wobei sich die pulverförmige Zusammensetzung in eine Flüssigkeit umwandelt,
- die keratinhaltigen Fasern
- vor, nach oder während des Auftragens der pulverförmigen Zusammensetzung und/oder
- vor, nach oder während der mechanischen Belastung der pulverförmige Zusammensetzung
in Form gebracht werden,
- die Form der keratinhaltigen Fasern durch die in besagte Flüssigkeit umgewandelte pulverförmige Zusammensetzung temporär fixiert wird.

## Claims

1. Process for manufacturing a powdered composition wherein
- a liquid, aqueous phase and hydrophobised metal oxide powder are mixed together with stirring, and a powdered composition containing core-shell particles is formed as the resulting mixture whose shell comprises hydrophobised metal oxide powder and whose core contains a liquid, aqueous phase,
- to the resulting powdered composition is added at least one film-forming and/or setting polymer in the form of a solid and is blended with stirring to a powdered composition,
**characterised in that** said film-forming and/or setting polymer is selected from at least one polymer of the group that is formed from copolymers of acrylic acid, (C₁ to C₄) alkyl acrylate, *N*-(C₄-alkyl)aminoethyl methacrylate and *N*-(C₈-alkyl)acrylamide.

2. Process according to claim 1, **characterised in that** the hydrophobised metal oxide of the hydrophobised metal oxide powder is selected from at least one member of the group that is formed from hydrophobised silicates, hydrophobised aluminium silicates, hydrophobised titanium dioxide as well as hydrophobised silicon dioxide.

3. Process according to one of claims 1 or 2, **characterised in that** hydrophobised metal oxide powder is comprised in an amount of 0.5 wt % to 30 wt % based on the weight of the end product of the process.

4. Process according to one of claims 1 to 3, **characterised in that** the hydrophobised metal oxide powder is selected from silica silylates.

5. Process according to one of claims 1 to 4, **characterised in that** the liquid, aqueous phase of the core comprises an aqueous solvent in an amount of 70 wt % to 90 wt % based on the weight of the end product of the process.

6. Process according to one of claims 1 to 5, **characterised in that** said film-forming and/or setting polymer is comprised in the form of particles in an amount of 0.01 wt % to 15 wt % based on the weight of the total powdered composition.

7. Process according to one of claims 1 to 6, **characterised in that** the liquid, aqueous phase of the core has a pH of 2 to 7, preferably 4 to 6.

8. Process according to one of claims 1 to 7, **characterised in that** the liquid, aqueous phase of the core additionally comprises at least one organic carboxylic acid with 2 to 7 carbon atoms.

9. Powdered composition obtainable by a process according to one of claims 1 to 8.

10. Use of a powdered composition according to claim 9 for the temporary shaping of keratin-containing fibres, in particular human hair.

11. Method for the temporary shaping of keratin-containing fibres, in particular human hair, in which method
- a powdered composition according to claim 9 is exposed to a mechanical stress before, during or after application onto the keratin-containing fibres, whereby the powdered composition is transformed into a liquid,
- the keratin-containing fibres
- before, during or after application of the powdered composition and/or
- before, during or after the mechanical stressing of the powdered composition
are brought into shape,
- the shape of the keratin-containing fibres is temporarily fixed by the powdered composition transformed into said liquid.

## Revendications

1. Procédé pour la préparation d'une composition pulvérulente, dans lequel :
- on mélange tout en agitant une phase liquide aqueuse et une poudre d'oxyde métallique rendue hydrophobe et on obtient comme mélange résultant une composition pulvérulente comprenant des particules du type à noyau-enveloppe dont l'enveloppe contient de la poudre d'oxyde métallique rendue hydrophobe, et dont le noyau comprend une phase liquide aqueuse ;
- à la composition pulvérulente ainsi obtenue, on ajoute au moins un polymère filmogène et/ou consolidant sous la forme d'une substance solide et on mélange tout en agitant pour obtenir une composition pulvérulente,
**caractérisé en ce que** ledit polymère filmogène et/ou consolidant est choisi parmi au moins un polymère du groupe qui est formé par des copolymères d'acide acrylique, d'acrylate d'alkyle en C₁-C₄, de méthacrylate de N-(alkyl en C₄)aminoéthyle et de N-(alkyl en C₈)acrylamide.

2. Procédé selon la revendication 1, **caractérisé en ce que** l'oxyde métallique rendu hydrophobe de la poudre d'oxyde métallique rendue hydrophobe est choisi parmi au moins un représentant du groupe qui est formé par des silicates rendus hydrophobes, des silicates d'aluminium rendus hydrophobes, du dioxyde de titane rendu hydrophobe et du dioxyde de silicium rendu hydrophobe.

3. Procédé selon l'une quelconque des revendications 1 ou 2, **caractérisé en ce que** la poudre d'oxyde métallique rendue hydrophobe est contenue en une quantité de 0,5 % en poids à 30 % en poids, rapportés au poids du produit final issu du procédé.

4. Procédé selon l'une quelconque des revendications 1 à 3, **caractérisé en ce que** la poudre d'oxyde métallique rendue hydrophobe est choisie parmi du Silica Silylate.

5. Procédé selon l'une quelconque des revendications 1 à 4, **caractérisé en ce que** la phase liquide aqueuse du noyau contient un solvant aqueux en une quantité de 70 % en poids à 90 % en poids, rapportés au poids du produit final issu du procédé.

6. Procédé selon l'une quelconque des revendications 1 à 5, **caractérisé en ce que** ledit polymère filmogène et/ou consolidant est contenu sous la forme de particules en une quantité de 0,01 % en poids à 15 % en poids, rapportés au poids de la composition pulvérulente totale.

7. Procédé selon l'une quelconque des revendications 1 à 6, **caractérisé en ce que** la phase liquide aqueuse du noyau possède une valeur de pH de 2 à 7, de préférence de 4 à 6.

8. Procédé selon l'une quelconque des revendications 1 à 7, **caractérisé en ce que** la phase liquide aqueuse du noyau contient en outre au moins un acide carboxylique organique contenant de 2 à 7 atomes de carbone.

9. Composition pulvérulente que l'on obtient via un procédé selon l'une quelconque des revendications 1 à 8.

10. Utilisation d'une composition pulvérulente selon la revendication 9, pour la permanente temporaire de fibres kératiniques, en particulier de cheveux humains.

11. Procédé pour la permanente temporaire de fibres kératiniques, en particulier de cheveux humains, dans lequel :
- on expose une composition pulvérulente selon la revendication 9, avant, pendant ou après l'application sur les fibres kératiniques, à une sollicitation mécanique, donnant lieu à la transformation de la composition pulvérulente en un liquide ;
- on donne une forme aux fibres kératiniques
- avant, après ou pendant l'application de la composition pulvérulente
et/ou
- avant, après ou pendant la sollicitation mécanique de la composition pulvérulente ;
- on fixe temporairement la forme des fibres kératiniques via la composition pulvérulente transformée pour obtenir ledit liquide.
